# EUROPEAN PATENT SPECIFICATION

(11) **EP 1 778 751 B1**
(45) Date of publication and mention of the grant of the patent: **01.03.2017**
(21) Application number: 05784971.3
(22) Date of filing: 12.08.2005
(51) Int. Cl.: C07H 13/06, C08F 20/20

(54) **CARBOHYDRATE BASED ALKYDS**
ALKYDHARZE AUF KOHLENHYDRATBASIS
ALKYDES A BASE DE CARBOHYDRATES

(30) Priority: 12.08.2004 US 600976 P
(43) Date of publication of application: 02.05.2007
(73) Proprietor: Sherwin-Williams Luxembourg Investment Management Company S.à r.l., 1857 Luxembourg (LU)
(72) Inventor: TOMKO, Richard, F., North Olmsted, OH 44070 (US); HENDKING, Wayne, T., Twinsburg, OH 44236 (US); MACKULIN, Peter, J., Jr., North Olmsted, OH 44070 (US); SIY, Jose Rullen R., Parma Heights, OH 44130 (US); GLINSKI, Charles, S., Painesville, OH 44077 (US)
(74) Representative: Heinemann, Monica
(86) International application number: PCT/US2005/028654
(87) International publication number: WO 2006/020818

(56) References cited:
- WO-A-92/21765
- WO-A-98/51694
- US-A- 3 356 652
- POTIER, P. ET AL.: "Lipase-catalysed selective synthesis of sucrose mixed diesters" SYNTHESIS, 2001, pages 458-462, XP002387146
- BOBALEK, E.G. ET AL.: "Drying oil esters of sucrose" OFFICIAL DIGEST, 1961, pages 453-468, XP009068363

## Description

This application claims the benefit of U.S. provisional patent application 60/600976 filed August 12, 2004.

This invention relates to alkyd compositions comprising esterified carbohydrates. The carbohydrate-based alkyds have both pendent fatty acid residues and pendent (meth)acrylate groups. As used herein, the term "fatty acid residue" means the group R of a fatty acid R'-COOH, or of the corresponding fatty acid derivative, wherein the fatty acid residue is containing linoleic, linolenic or oleic acid. The R' group will typically be pendent from the backbone through an ester linkage. The term "(meth)acrylate" is used herein to mean either acrylate or methacrylate functionality. The alkyds can be useful in coatings, inks, adhesives, plastics and other applications. The alkyds may be utilized by themselves as the sole film former, or, in other embodiments of this invention, in combination with a variety of reactive diluents, monomers, oligomers or polymers, and cured by any acceptable method, such as, for example, oxidative curing at ambient or elevated temperatures, or high energy curing utilizing visible light, UV or electron beam radiation or combinations thereof. In one useful application, the alkyds described herein can provide low viscosity coatings having excellent cure and hardness when applied to any of a variety of substrates, including wood substrates.
Drying oil esters of sucrose, such as sucrose linoleate, are described in "Bobalek et al, Official Digest, April, 1961, pages 453 to 468. These esters do not contain pendent (meth)acrylate groups.

### BRIEF SUMMARY OF THE INVENTION

This invention relates to alkyds having pendent (meth)acrylate groups, said alkyd comprising an esterified carbohydrate, said esterified carbohydrate being from about 20 to about 100 percent esterified or otherwise reacted based upon the total hydroxyl equivalency of the underlying carbohydrate. In one embodiment, from about 3 percent to about 97 percent, on average, of the initial hydroxyl equivalency is consumed by reaction with saturated or unsaturated fatty acids, wherein the fatty acid residue is containing linoleic, linolenic or oleic acid, or reactive derivatives thereof having from about 6 to about 30, and generally 6 to about 18, carbon atoms, and wherein about 3 percent to about 97 percent, on average, of the initial hydroxyl equivalency is consumed by reaction with reactants having a (meth)acrylate moiety and a reactive moiety capable of esterification or other reaction with the remaining hydroxyls, or reactive derivative functional groups thereof.

As used herein, the term "carbohydrates" generally includes, but is not limited to, polyhydroxyl aldehydes, polyhydroxy ketones, or compounds that can be hydrolyzed to them. Representative naturally occurring carbohydrates include sugars, starches and fibers such as cellulose. For some applications of this invention, the carbohydrate substances are selected from the group consisting of monosaccharides, oligosaccharides, and polysaccharides having from 2 to about 15 saccharide units per molecule. As used herein, the term "carbohydrates" also includes those substances obtained from saccharides or other carbohydrates by reduction of the carbonyl group such as alditols, by oxidation of one or more terminal groups to carboxylic acids, or by replacement of one or more hydroxyl groups by a hydrogen atom, an amino group, a thiol group or other heteroatomic groups, as well as reactive derivatives of these compounds. Although sugar alcohols are not carbohydrates in a strict sense, the naturally occurring sugar alcohols are so closely related to the carbohydrates that they are also practical for use herein and are included in the term "carbohydrates" for purposes of this invention. The sugar alcohols most widely distributed in nature and suitable for use herein include sorbitol, mannitol and galactitol. The carbohydrates provide an effective backbone for the alkyds of this invention and have the advantage of being a renewable resource as opposed to petroleum-based raw materials.

In one embodiment, this invention relates to coatings comprising the alkyds of this invention, optionally in combination with other air-drying compositions, such as conventional alkyds, and/or optionally in combination with ethylenically unsaturated compounds which are copolymerizable with the (meth)acrylate functional alkyd. The alkyds of this invention can, if desired, be cured by multiple curing mechanisms such as, for example, those involving both oxidative drying and free radical or high energy cure, or those involving oxidative cure and chemical crosslinking, or by any of these curing methods alone.

In one embodiment, this invention relates to curable compositions comprising:
(i) an esterified carbohydrate alkyd having pendent (meth)acrylate functionality;
(ii) optionally, at least one other ethylenically unsaturated radiation polymerizable reactant; and
(iii) optionally, at least one other air drying polymer; and
(iv) optionally, at least one chemical crosslinker reactive with the alkyd; and
(v) at least one catalyst or initiator for initiating cure.

The coatings can be applied to any substrate and then cured by any suitable method.

### DETAILED DESCRIPTION OF THE INVENTION

The alkyds of this invention have both pendant fatty acid residues as defined in claim 1 and pendant (meth)acrylate groups. The alkyds can conveniently be prepared by reaction of a carbohydrate, or a reactive derivative thereof, with (i) fatty acids or derivatives thereof and also with (ii) reactants having at least one (meth)acrylate moiety and at least one moiety reactive with the carbohydrate or reactive derivative thereof.

### 1. Carbohydrate substances.

Useful carbohydrate starting materials for the alkyds of this invention include carbohydrates having a hydroxyl group or a reactive derivative thereof such as a lower acyl ester or acid group. By "reactive derivatives thereof" is meant a derivative of the carbohydrate wherein at least some of the hydroxyl groups are replaced by other groups which are reactive with hydroxyl, ester, epoxy or acid groups. Such reactive groups on the carbohydrate would include ester, especially lower acyl ester, and acid groups. For example, some of the hydroxyl groups of the carbohydrate can be converted to lower acyl esters through an alcoholysis reaction with an ester such as methyl acetate, or converted into acid groups by reaction with an anhydride such as succinic anhydride. Useful carbohydrates include monosaccharides, for example, mannose, galactose, arabinose, xylose, ribose, apiose, rhamnose, psicose, fructose, sorbose, tagatose, ribulose, xylulose, and erythirulose. Oligosaccharides suitable for use herein include, for example, maltose, kojibiose, nigerose, cellobiose, lactose, melibiose, gentiobiose, turanose, rutinose, trehalose, sucrose and raffinose. Polysaccharides suitable for use herein include, for example, amylose, glycogen, cellulose, chitin, inulin, agarose, zylans, mannan and galactans. The carbohydrate starting materials can also be chain extended if desired by reaction, for example with polyisocyanates, or di or polyesters to increase molecular weight. Due to cost, availability and other considerations, one frequently useful carbohydrate is sucrose.

### 2. Preparation of alkyds by reaction with fatty acids and derivatives thereof.

These carbohydrates can be reacted with fatty acids, or fatty acid derivatives, as described herein, to produce alkyds having a carbohydrate backbone with some of the original carbohydrate hydroxyl equivalency esterified or otherwise reacted with unsaturated fatty acids, or fatty acid derivatives. As used herein, the term "fatty acid derivative" means a reactive derivative of a fatty acid, such as the acid chloride, anhydride, or ester thereof, including fatty acid oils such as triglycerides. The fatty acid chains of the fatty acids or fatty acid derivatives can be branched, linear, saturated, unsaturated, hydrogenated, unhydrogenated, or mixtures thereof. In one embodiment it is preferred that at least some of the fatty acid chains are unsaturated drying oil or semi-drying oil chains. According to the invention, the fatty acids are those containing linoleic, linolenic and/or oleic acids. If desired, the acids can be converted to their corresponding reactive derivatives such as esters, acid chlorides, or acid anhydrides. The acid chlorides are conveniently prepared by reaction of the fatty acid with a suitable chloride, such as thionyl chloride. Fatty acid anhydrides can be prepared by methods well known in the art such as reaction of the corresponding acid with a dehydrating agent such as acetic anhydride. Suitable fatty oils include sunflower oil, canola oil, dehydrated castor oil, coconut oil, corn oil, cottonseed oil, fish oil, linseed oil, oiticica oil, soya oil, tung oil, tall oil, castor oil, palm oil, safflower oil, blends thereof, and the like.

In general, the alkyds can be produced by the reaction of the carbohydrate and the fatty acid, fatty acid anhydride, fatty acid ester or oil by any method known in the art to produce the desired extent of reaction of the underlying carbohydrate hydroxyl groups. For example, U.S. 5,231,199 teaches synthesis of carbohydrate based alkyds by the reaction of the carbohydrate with a fatty acid lower alkyl ester, such as e.g. an alkali metal hydroxide or carbonate, preferably in the presence of a stripping agent such as hexane or an inert gas. Other processes for the production of similar alkyds are taught in U.S. 3,963,699, U.S. 4,517,360, and U.S. 4,518772. U.S. 5,158,796 references U.S. 2,831,854, and teaches alkyds of carbohydrates having the majority, and preferably at least about 80% of their hydroxyl groups esterified by fatty acids by such reaction mechanisms as transesterification of the carbohydrate with methyl, ethyl or glycerol fatty acid esters using a variety of catalysts; by acylation of the carbohydrate with a fatty acid chloride; or by acylation with the fatty acid itself. Generally, the reaction to produce carbohydrate based alkyds can be conducted neat, or in a suitable solvent, including exempt solvents, or in the presence of a diluent.

Another useful method for preparing the alkyds is taught in U.S. published application 2002/0143137 A1 to Howle et. al. and involves the reaction of polyols such as saccharides with fatty acid esters in the presence of a basic catalyst, followed by separation of any remaining excess fatty acid ester once the desired degree of transesterification (alcoholysis) has taken place. The desired degree of esterification can be controlled by adjusting the amount of excess fatty acid ester and by monitoring the time and extent of reaction so that less than all the available hydroxyl groups are converted to esters. Another useful approach to prepare the fatty acid alkyds having hydroxyl groups which remain after the esterification is taught in U.S.2003/0229224 A1 to Schaefer et. al. In this process, a highly esterified fatty acid polyol polyester and a lesser (or non) esterified polyol are admixed to produce a first reaction product having an average degree of esterification less than that of the highly esterified starting material, followed by reaction with an additional polyol portion to further reduce the average level of esterification per carbohydrate molecule.

For purposes of this invention, the fatty acid or fatty acid derivative can be reacted with the carbohydrate in an amount to convert, on average, from 3% to 97% of the hydroxyl groups of the carbohydrate. Commercially available fatty acid modified carbohydrate based alkyds include the Sefose® products produced by Proctor and Gamble.

If desired, the carbohydrate based alkyd could be chain extended to increase its molecular weight. The chain extension is readily accomplished by reaction with a di-functional material which is reactive with the pendant groups of the carbohydrate or carbohydrate based alkyd. The chain extension can be done before or after the reaction of the carbohydrate with the fatty acid or before or after the reaction which incorporates the (meth)acrylic functionality. For example, if the carbohydrate or alkyd has remaining active hydrogen groups, such as hydroxyl groups, the chain extension can be by reaction with a poly or diisocyanate, optionally in the presence of a suitable catalyst such as a tertiary amine or a metal compound such as dibutyl tin dilaurate. Representative diisocyanates include isophorone diisocyanate, tetramethylene diisocyanate, hexamethylene diisocyanate, 1,4-naphthalene diisocyanate, and 2,4- or 2,6-toluene diisocyanates. Alternatively, di or poly functional acids, esters, or silanols or silanes, such as Silres® SY231, or Dow Corning 3037 or Z6018, or mercaptopropyl trimethoxysilane (General Electric silicone A-189) could also be utilized. If the carbohydrate has pendant ester groups, such as lower acyl esters, the chain extension can be accomplished representatively by transesterification with a diester. The chain extender should be added in only such amount as appropriate to obtain the desired molecular weight. For low viscosity, high solid applications, preferably the final number average molecular weight of the chain extended carbohydrate alkyd would typically be less than about 5,000.

### 3. Reactants having a (meth)acrylic moiety and a moiety reactive with the carbohydrate.

Reactants having at least one (meth)acrylic moiety and at least one moiety reactive with the carbohydrate or reactive derivative thereof are well-known in the art and in general are derivatives of acrylic or methacrylic acid. Moieties which are reactive with the carbohydrate or reactive derivatives thereof include moieties having carboxyl, hydroxyl, isocyanato, silane, or epoxy groups. Representative reactants include: acrylic acid, methacrylic acid, and (meth)acrylates of monohydric alcohols, and similar (meth)acrylate acid derivatives, such as methyl methacrylate, 2-ethylhexyl acrylate, n-butyl acrylate and lauryl methacrylate. Acrylic anhydride, methacrylic anhydride, and the acid chlorides of acrylic or methacrylic based acids are also practical. Useful isocyanate functional monomers include isocyanatoethyl and isocyanatopropyl (meth)acrylates and 2,2'-dimethyl-1,3-isopropenyl benzyl isocyanate (also known as dimethyl meta-isopropenyl benzyl isocyanate). Useful epoxy functional monomers include glycidyl (meth)acrylates. Useful hydroxyl functional monomers include 2-hydroxyethyl acrylate, 2-hydroxyethyl methacrylate, 2-hydroxypropyl acrylate, 4-hydroxybutyl methacrylate, 2-hydroxypropyl methacrylate, 3-hydroxybutyl acrylate, 4-hydroxypentyl acrylate, 3-hydroxybutyl methacrylate, 2-hydroxyethyl chloroacrylate, diethylene glycol methacrylate, tetraethylene glycol acrylate, and caprolactone adducts with 1 to 5 caprolactone units, such as Tone® M100 (trademark of Dow Chemical's hydroxyfunctional acrylic caprolactone adduct believed to be the reaction product of 1 mole of hydroxy ethyl acrylate and 2 moles of caprolactone).

The (meth)acrylate functional materials can be reacted with the carbohydrate or carbohydrate based alkyd by any of the methods well known in the art. For example, a hydroxyl functional carbohydrate or carbohydrate based alkyd could be reacted with an appropriate stoichiometric amount of methacrylic acid chloride at temperatures typically ranging up to about 50°C in the presence of a base such as triethyl amine. After the reaction is complete, the triethyl amine salt can be filtered to leave the desired product. In an alternative approach, methacrylic anhydride can be admixed with the alkyd or carbohydrate in the presence of a suitable catalyst and reacted at temperatures typically ranging up to about 200°C until the desired degree of reaction is attained. The methacrylic acid by-product can be removed during the reaction by an air-sparge, or could be removed under vacuum, or subsequently stripped by any conventional method such as by use of a thin film or wiped film evaporator. Alternatively, the remaining methacrylic acid monomer, optionally along with other copolymerizable monomers, could be polymerized into the methacrylate functional alkyd to create an acrylic modified alkyd by polymerizing the monomers and the methacrylate functional alkyd in the presence of a suitable initiator.

Since the order of reaction of the fatty acid or methacrylate material with the carbohydrate is not critical, either reaction could take place first, or alternatively, both could take place simultaneously, such as by reacting an appropriate mixture of e.g. the methyl ester of a fatty acid and a lower alkyl ester of a (meth)acrylic acid with the hydroxy functional carbohydrate under alcoholysis reaction conditions. Another useful approach to prepare the (meth)acrylate functional alkyds of this invention is to react the hydroxy functional carbohydrate or carbohydrate-based alkyd directly with an epoxy functional (meth)acrylate such as glycidyl methacrylate in the presence of an acid or base catalyst. In another representative approach, the carbohydrate or carbohydrate based alkyd can be converted into an acid functional compound by reaction of hydroxyl groups with succinic anhydride to obtain acid functionality, and the acid functional material could subsequently be reacted with glycidyl methacrylate.

Another approach is the transesterification (alcoholysis) of the hydroxyl groups remaining on the alkyd or the carbohydrate with lower alkyl (meth)acrylic esters. These transesterification reactions can be conducted solvent free or in a suitable solvent such as dimethylformamide or pyridine. The reaction mixture is heated to temperatures up to about 180°C, and generally about 120°C to 150°C in the presence of a transesterification catalyst. The reaction is generally conducted under reflux and/or vacuum to remove low molecular weight by-products and may include a soap or other emulsifying or compatabilizing agent.

In another approach, an excess of diisocyanate could be reacted with the hydroxyl groups of the alkyd or carbohydrate, and the isocyanate functional product could be reacted a hydroxy or amine functional (meth)acrylate material. Another exemplary approach is to prepare the lower acyl ester of the carbohydrate and react that with the lower alkyl esters of the fatty acids and methacrylate esters through a transesterification reaction. The actual method of preparation of the final alkyd product having both pendant fatty acid residues and pendant (meth)acrylate functionality is not critical to this invention. Mixtures of various fatty acid derivatives and various (meth)acrylate starting materials are also practical. For some applications, it is convenient to conduct the reaction of the (meth)acrylic material and the alkyd or carbohydrate in the presence of an inhibitor such as a phenothiazine or a phenolic inhibitor such as BHT (commonly known as butylated hydroxy toluene) or Irganox® 1076 (octadecyl-3-5-di-tert-butyl-4-hydroxyhydrocinnamate), to minimize premature polymerization of the (meth)acrylic groups. The inhibitor can also be added after the (meth)acrylic functional alkyd has been prepared to enhance shelf life. For purposes of this invention, the (meth)acrylate functional material can generally be reacted with the carbohydrate in an amount to convert from 3% to 97% of the initial hydroxyl groups of the carbohydrate. The addition of the pendent (meth)acrylate group to the alkyd can provide a final alkyd having additional cure mechanisms, low viscosity, and enhanced cure and hardness. For certain low VOC applications, it is often useful to produce the alkyds of this invention with a viscosity at 99% NVM of less than 10,000 centipoise, and frequently less than 5,000 centipoise, when measured at 25°C using a Brookfield LVT #3 spindle at 30 rpm. The very low viscosity of these alkyds makes them especially useful as reactive diluents and/or film forming polymers in coatings and other applications.

### 4. Combinations of the (meth)acrylic alkyds with other materials.

The alkyds of this invention are particularly versatile due to their relatively low viscosity, and due to having both fatty acid and (meth)acrylate functionality for cure and other performance properties. The alkyds can be used alone or in combination with other materials to obtain a variety of desirable properties. For many applications, the alkyds of this invention will typically comprise at least 5%, and normally 5-100% by weight of all the materials comprising the film forming polymerizable components. In one embodiment, the alkyd of this invention will comprise between about 20% and 90%, and in another embodiment, between 30 and 70% of the film forming polymerizable components of the combination.

The remaining materials could be other polymers such as other alkyds (including alkyds of carbohydrates which do not have pendant (meth)acrylic functionality), polyesters, epoxies, vinyl resins, phenolics, fatty phenolics, acrylics, silicones, polyurethanes, polyureas, polyolefins, reactive diluents, natural oils, and mixtures thereof. When used alone, or in combination with other film forming polymers, the alkyds of this invention could also be combined with crosslinking agents such as polyisocyanates, melamines, ureas or other crosslinkers. The alkyds of this invention can utilize one or more of multiple curing mechanisms including chemical crosslinking, oxidative cure, photo initiated cures, and high energy cures.

For air drying cures, the alkyds will typically contain driers to accelerate the air dry. Typical driers are well known in the art and include various salts of cobalt, zirconium, calcium, zinc, lead, iron, cerium, neodymium, aluminum and manganese. Cobalt driers are frequently useful and combinations of driers are frequently used. Frequently the driers are used as octoates or naphthenates and are typically incorporated in an amount from 0.003-0.75% based on the weight of the air-drying materials. The coatings could be air dried at ambient temperatures or force dried at temperatures ranging up to 300°F or higher.

If desired, solvents can be incorporated into the coating systems, but due to the relatively low molecular weight and low viscosity of the alkyds of this invention, the solvent requirements will be greatly reduced compared to many prior art alkyds. If desired, suitable solvents include mineral spirits, ketones, aromatic hydrocarbons, esters such as t-butyl acetate and Oxsol 100 (available from Kowa America).

Due to the (meth)acrylate functionality incorporated into the alkyds, they can be conveniently combined with other unsaturated, polymerizable materials such as ethylenically unsaturated monomers, oligomers or polymers and cured by exposure to free radicals, visible light, high energy such as ultra violet or electron beam, or by other methods. When used in such a combination, the alkyd will typically be present at a level of 5 to about 100% by weight of all the polymerizable materials.

Representative monomers include 4-hydroxypentyl acrylate, diethylene glycol methacrylate, tetra ethylene glycol acrylate, para-vinyl benzyl alcohol, etc.; esters of acrylic, methacrylic, crotonic, tiglic, or other unsaturated acids such as: methyl acrylate, ethyl acrylate, propyl acrylate, isopropyl acrylate, butyl acrylate, isobutyl acrylate, ethylhexyl acrylate, amyl acrylate, 3,5,5-trimethylhexyl acrylate, methyl methacrylate, ethyl methacrylate, propyl methacrylate, dimethylaminoethyl methacrylate, isobornyl methacrylate, t-butyl methacrylate, gamma-methacryloxypropyltrimethoxy silane (Siliquest® A-174) ethyl tiglate, methyl crotonate, ethyl crotonate; vinyl compounds such as vinyl acetate, vinyl propionate, vinyl butyrate, vinyl isobutyrate, vinyl benzoate, and vinyl toluene; styrene based materials such as styrene and alpha methyl styrene; allyl compounds such as allyl glycidyl ether, allyl acetate, and allyl benzoate; and other copolymerizable unsaturated monomers such as isopropenyl acetate, isopropenyl isobutyrate, dimethyl and other maleates, and polyunsaturated monomers such as di-, tri-, and poly- (meth)acrylates such as, for example, ethylene glycol diacrylate, 1,3-propylene glycol diacrylate, 1,4- butanediol diacrylate, 1,4-cyclohexane diacrylate, 1,6-hexanediol diacrylate, neopentyl glycol diacrylate, 1,5-pentanediol diacrylate, 1,8- octanediol diacrylate, trimethylol propane triacrylate, pentaerythritol triacrylate, pentaerythritol tetraacrylate, and dipentaerythritol pentaacrylate, etc. Oligomers and unsaturated polymers such as unsaturated polyesters, and multi-functional polymeric (meth)acrylates such as urethane poly(meth)acrylates can also be utilized. As used herein, the term "film-forming polymer" means any polymeric material that can form a film by cure or by evaporation or any carrier or solvent.

If high energy cure is desired, the polymerization of the curable compositions can be effected with, for example, visible light, UV light or electron beam or visible light and will typically include a suitable initiator for this cure. Polymerization is especially preferred by exposure to UV light. Suitable initiators include: acylphosphine oxides such as 2,4,6-trimethylbenzoyl diphenyl phosphine oxide, benzoyldiethoxyphosphine oxide and 2,4,6-trimethylbenzoyl dinaphthyl phosphine oxide, phenylbis(2,4,6-trimethylbenzoyl) phosphine oxide, commercially available as IRGACURE® 819; Michler's ketone; benzil; 2-chlorothioxanthone; 2,4-diethylthioxanthone; 2,4-diisopropylthioxanthone; 2-benzyl-2-(dimethylamino)-1-(4-(4-morpholinyl)phenyl)-1-butanone, commercially available as IRGACURE® 369; camphoroquinone and mixtures thereof. Additional initiators include: acyloin and derivatives thereof, such as benzoin, benzoin methyl ether, benzoin ethyl ether, benzoin isopropyl ether commercially available as VICURE-30 from Stauffer Chemicals, benzoin isobutyl ether commercially available as TRIGONAL-14 from Noury, and methylbenzoin; diketones such as diacetyl; aromatic diketones such as anthraquinone; phenones such as acetophenone, 2,2,2-tribromo-1-phenylethanone, 2,2-diethoxyacetophenone, 2,2-dimethoxy-2-phenylacetophenone commercially available as IRGACURE® 651 from Ciba Geigy, 2,2,2-tribromo-1-(2-nitrophenyl)ethanone, benzophenone, and 4,4-bis(dimethylamino)benzophenone, 2-hydroxy-2-methyl-1-phenyl-propan-1-one commercially available as DAROCUR® 1173 from Ciba Geigy, hydroxycyclohexyl phenyl ketone commercially available as IRGACURE® 184 from Ciba Geigy, 2-methyl-1-(4-(methylthio)phenyl)-2-morpholino-propanone-1 commercially available as IRGACURE® 907 from Ciba Geigy, 2,2-dichloro-1-(4 phenoxyphenyl)ethanone commercially available as SANDORAY-1000 from Sandoz, chlorinated benzophenone aryl ketone commercially available as FI-4 from Eastman Kodak, and α-isobutyl-α-phenyl acetophenone commercially available as VICURE-10 from Stauffer Chemicals. Visible light initiators include benzil, benzoin, benzoin methyl ether, DL-camphorquinone (CQ), benzil diketones and acylphosphine oxides.

Peroxide initiators such as hydroperoxides, acyl peroxides, keto peroxides, dialkyl peroxides, peroxyesters, and peroxyketals also can be utilized and selected examples include cumene hydroperoxide, methyl ethyl ketone peroxide, acetal acetone peroxide, tertiary butyl perbenzoate, dicumyl peroxide, benzoyl peroxide, cyclohexanol peroxide, or methyl isobutyl ketone peroxide, and combinations thereof. Azo compounds, such as azobisisobutyronitrile, or 2-t-butylazo-2- cyanobutane could also be utilized. The peroxide initiators could be activated by heat, radiation, or oxidation/reduction.

The total amount of initiator present in the curable coating composition is generally in the range of about 0.05-10% by weight of the total composition, preferably between about 0.2% and about 7%. The high energy curing, if desired, of the coatings of this invention is completed by sufficient exposure to radiation to obtain the desired degree of cure. The curing is accomplished by exposing the coating to suitable radiation sources such as visible light, mercury lamps, xenon lamps, argon lamps, metal halide lamps, electron beams, or other device to produce the radiation. The coatings can also be cured in air with only photoinitiators if desired.

The alkyds of this invention can also be formulated to include additives which do not adversely effect curing of the coating. Suitable amounts of pigments, solvents, thixotropes, flow control additives, diluents, light stabilizers such as Tinuvin® 5060, fire startdants, smoke suppressants, decolorizing additives, and other materials can be utilized.

The curable compositions of this invention comprise the (meth)acrylate functional alkyds, either alone or in combination with other unsaturated materials, other film forming polymers, crosslinkers and, if desired, appropriate driers, initiators and catalysts. The curable compositions may typically be applied to any substrate such as metal, plastic, wood, glass, masonry, porous stone, synthetic fibers, etc. by brushing, dipping, roll coating, flow coating, vacuum coating, spraying or other method conventionally employed in the art. The curable composition will be applied to the substrate typically to provide a dry film thickness of at least about 0.1 mil and will typically be applied between 0.1 and 10 mils dry film thickness. It is frequently useful to utilize the (meth)acrylate functional alkyds of this invention to prepare pigmented coatings having a viscosity of less than 150 Krebs Units at a VOC of less than 150 grams/liters. Representative pigments include talcs, silicas, barites, titanium dioxide, zinc oxide, carbon black, phthalocyanine blue, and synthetic polymeric pigments. The coatings may also typically include one or more organic solvents.

The following examples have been selected to illustrate specific embodiments and practices of advantage to a more complete understanding of the invention. Unless otherwise stated, "parts" means parts-by-weight and "percent" is percent-by-weight.

### EXAMPLE 1

### A representative example of a methacrylated alkyd was prepared as follows:

A reaction vessel equipped with a mechanical stirrer, thermocouple, and air inlet was charged with 3000 parts of a 60 hydroxyl number sucrose soya fatty acid ester (having an average of about 6 of the 8 sucrose hydroxyl groups esterified by reaction with a lower alkyl ester of the fatty acid) and heated to 170°C. 1 part N,N dimethylbenzylamine, 520 parts of methacrylic anhydride, and 0.12 parts butyrated hydroxyl toluene (BHT) were added and the mixture stirred and maintained at 170°C. Air was bubbled through the reaction mixture to help remove the methacrylic acid by-product into a condenser equipped with a sodium hydroxide solution reservoir. Air was continuously blown through the reaction mixture and distillation was continued for about two hours. The resulting resin had an Acid Value of 4-7, Brookfield Viscosity LVT#3 spindle @ 30 rpm and 25°C of less than 2600cps and NVM (Non Volatile Material) greater than 99.5%. Alternative methods of removing the by-products, such as by thin or wiped film evaporator, or distillation under reduced pressure are also practical.

### EXAMPLE 2

### Methacrylation of sucrose esters by reaction with succinic anhydride followed by glycidyl methacrylate.

A reaction vessel was charged with 1000 parts of the hydroxy functional sucrose soya ester starting material of Example 1, 85.5 parts succinic anhydride and 55 parts methyl propyl ketone. The mixture was heated under nitrogen to 120°C and 0.1 part N,N-dimethylbenzylamine was added and the reaction maintained at 120°C for 2 hours. A mixture of 121.7 parts glycidylmethacrylate and 0.1 part BHT was added over a thirty minute period. The reaction was maintained at temperature for an additional hour and then a distillation head/condenser assembly for vacuum distillation was added and the pressure was reduced to 400 millibar and held for one hour and then continued under reduced pressure until distillation was complete. The resin had an NVM of 98.5% and a Brookfield LVT viscosity at room temperature #3 spindle at 30 rpm of 3824 cps.

### EXAMPLE 3

### Chain Extension of Sucrose Soya ester with IPDI and methacrylation with Methacrylic anhydride.

A reaction vessel was charged with 1,200 parts of the hydroxy functional sucrose soya ester starting material of Example 1, 0.1 part of dibutyl tin dilaurate and 71.4 parts of isophorone diisocyanate. The reaction mixture was heated to 170°C under a nitrogen blanket. The nitrogen was turned off and 0.1 part of N,N-dimethylbenzylamine, 104.2 parts methacrylic anhydride, and 0.04 parts BHT were added and the mixture was reheated to 170°C and an air line was used to bubble air for ten minutes. A vigorous air blow was then applied to distill methacrylic acid by-product into a sodium hydroxide trap. The distillation continued for one hour. The resulting resin had an Acid Value of 4.05, Brookfield viscosity LVT #3 spindle @ 30rpm and 25°C of 5360 cps and NVM of greater than 99.5%.

### EXAMPLE 4

### Methacrylation of sucrose esters by reaction with isophorone diisocyanate followed by hydroxyethylmethacrylate.

A two liter, four necked flask equipped with a mechanical stirrer, thermocouple, addition funnel and nitrogen inlet, was charged with 800 parts of the sucrose soya ester starting material of Example 1, 100 parts mineral spirits, and a catalytic amount of dibutyltin dilaurate. The mixture was heated to 40°C under nitrogen, and 185 parts of isophoronediisocyanate was added dropwise over 30-minutes and the mixture was heated for two additional hours at 40°C. The nitrogen inlet was replaced with a tube to bubble air into the resin and a mixture of 108 parts hydroxyethylmethacrylate and 0.1 part BHT (2,6-di-tert-butyl-4-methylphenol) was added through the addition funnel over 30-minutes. The mixture was heated at 40°C for an additional 120 minutes and then heated to 60°C for 180-minutes. The methacrylate functional alkyd product was characterized as having an NVM of 83%, and Brookfield LVT viscosity at room temperature #3 spindle at 12rpm of 4140 centipoise.

### EXAMPLE 5

### Reaction of Sucrose Alkyd with Glycidyl Methacrylate.

A four neck reaction flask, equipped with a mechanical stirrer and thermocouple, was charged with 1000 parts of the sucrose alkyd starting material of Example 1 and heated to 120°C. 0.1 part of N,N dimethylbenzylamine was added followed by dropwise addition of a mixture of 142 parts of glycidylmethacrylate and 0.1 parts of butyrated hydroxy toluene (2,6-di-tert-butyl-4-methylphenol) over 45 minutes. Heating was continued for six hours at 120°C. The resulting methacrylate functional alkyd resin had a Brookfield Viscosity LVT#3 spindle @ 30rpm and 25°C of 1236 cps and NVM of 99.2%.

### EXAMPLE 6

### Preparation of Methacrylated Sucrose Safflower Ester.

A reaction vessel equipped with a mechanical stirrer, thermocouple and air inlet was charged with 1425 parts of a 60 hydroxyl number sucrose safflower ester (having an average of about 6 of the 8 sucrose hydroxyl groups esterified by reaction with a lower alkyl ester of the fatty acid) and heated to about 170°C. 1 part N,N dimethylbenzylamine and 247.2 parts methacrylic anhydride were added to the mixture and the temperature maintained at 170°C. An air inlet was immersed into the resin and air was bubbled through the reaction mixture for about 10 minutes followed by a vigorous air sparge distillation of methacrylic acid for approximately two hours. The resulting methacrylated sucrose safflower alkyd had an acid value of 8.16, a Brookfield viscosity at room temperature LVT #3 spindle @ 30 rpm of 1500 cps and an NVM greater than 99.5%.

### EXAMPLE 7

### Paint Formula using Sucrose Soya Ester.

A representative paint sample was prepared by mixing the materials on a high speed mixer as follows:

| **Material** | **Parts** |
|---|---|
| Methacrylated Sucrose Soya Ester of Example 1 | 200.00 |
| Mineral Spirits | 55.00 |
| Bentone SD-1 Organophilic Clay | 7.50 |
| Lecithin | 12.30 |
| R-706 Titanium Dioxide | 205.00 |
| Calcium Carbonate | 426.99 |
| Dumacyl -100 Treated Silica from Hi-Mar Specialties | 20.00 |
| Polyvinyl chloride polymeric particle¹ | 42.30 |
| Methacrylated Sucrose Soya Ester of Example 1 | 146.00 |
| Sucrose Soya Ester² | 59.20 |
| Mineral Spirits | 50.00 |
| 12% Cobalt Octoate Catalyst | 1.48 |
| 10% Calcium Carboxylate | 7.91 |
| Neo-Cem 250³ | 9.88 |
| Dri-Rx HF⁴ | 0.79 |
| Methyl Ethyl Ketoxime | 3.95 |

| | |
|---|---|
| ¹ Geon 217 PVC particles from Poly One ² Non-methacrylated sucrose soya ester having an average of about 7.7 ester groups per sucrose molecule ³ 62% solution of Neodymium 2 ethyl hexanoate from OMG ⁴ Bipyridyl complexing agent from OMG | |

The coating exhibited a VOC of less than 150 g/l, a KU viscosity of 97, and excellent drying characteristics.

### EXAMPLE 8

A coating was prepared as described below by mixing the materials on a high speed mixer:

| **Material** | **Parts** |
|---|---|
| Glycidylmethacrylate soya sucrose ester of Example 2 | 200.00 |
| Aromatic Naphtha 100 | 20.00 |
| Bentone SD-1 | 2.88 |
| Lecithin, Soya | 7.70 |
| 10% Calcium Carboxylate | 4.00 |
| R-706 Titanium Dioxide | 180.00 |
| Talc Micronized Flaky | 15.00 |
| Calcium Carbonate | 210.00 |
| Halox CW 291 calcium borosilicate pigment | 15.00 |
| Glycidylmethacrylate soya sucrose ester of Example 2 | 341.50 |
| Aromatic Naphtha 100 | 37.94 |
| 12 % Cobalt Octoate Catalyst | 1.43 |
| 10% Calcium Carboxylate | 13.10 |
| Neo Cem 250 | 14.25 |
| Dri-RX HF | 0.86 |
| Methyl Ethyl Ketoxime | 4.00 |

The coating exhibited a VOC of only 97 g/l, a KU viscosity of 127, and excellent drying characteristics.

### EXAMPLE 9

The following coating was prepared as described below by admixing utilizing a high speed disperser.

| **Material** | **Parts** |
|---|---|
| Methacrylated Soya Sucrose Ester of Example 1 | 200.00 |
| Mineral Spirit | 10.00 |
| Bentone SD-1 | 3.00 |
| Lecithin, Soya | 10.00 |
| 10% Calcium Carboxylate | 4.00 |
| R-706 Titanium Dioxide | 280.00 |
| Calcium Carbonate Duramite | 25.0 |
| Calcium Carbonate Atomite | 80.00 |
| Methacrylated Soya Sucrose Ester of Example 1 | 280.00 |
| Versadil 1100¹ | 10.00 |
| Arylzene 7173² | 150.00 |
| Mineral Spirit | 23.76 |
| 12 % Cobalt Octoate Catalyst | 2.00 |
| 10% Calcium Carboxylate | 8.00 |
| Neo-Cem 250 | 15.00 |
| Dri-RX HF | 1.20 |
| Methyl Ethyl Ketoxime | 3.00 |

| | |
|---|---|
| ¹ unsaturated hydrocarbon reactive diluent from Neville Chemical ² hydroxy functional aromatic alkyd from Georgia Pacific. | |

The paint composition had a VOC of 97 g/l and a viscosity of 105 KU. The paint showed excellent drying characteristics.

### EXAMPLE 10

A coating was prepared as shown below by admixing with a high speed disperser.

| **Material** | **Parts** |
|---|---|
| Glycidylmethacrylate soya sucrose ester of Example 5 | 226.00 |
| Aromatic Naphtha 100 | 26.00 |
| Bentone SD-1 | 7.00 |
| Lecithin, Soya | 11.00 |
| R-706 Titanium Dioxide | 205.00 |
| Minex 4 Silica | 217.54 |
| Dumacil-100 Silica | 5.00 |
| Glycidylmethacrylate soya sucrose ester of Example 5 | 340.00 |
| Aromatic Naphtha | 36.00 |
| BYK 306 Flow Agent | 3.00 |
| 12 % Cobalt Octoate Catalyst | 2.12 |
| 10% Calcium Carboxylate | 11.32 |
| Neo Cem 250 | 14.15 |
| Dri-RX HF | 1.13 |
| Methyl Ethyl Ketoxime | 5.66 |

The coating exhibited a VOC of less than 100g/l and a KU viscosity of 86, and excellent drying characteristics.

### EXAMPLE 11

A clear coating capable of dual curing could be prepared by admixing:

| **Material** | **Parts** |
|---|---|
| 1,6 Hexanediol Diacrylate | 13.90 |
| Trimethylolpropane Triacrylate | 3.50 |
| Tripropylene Glycol Diacrylate | 30.00 |
| Methacrylated Carbohydrate Alkyd of Example 4 | 25.00 |
| Initiator | 3.85 |
| Amine Synergist² | 1.93 |
| 12% Cobalt Octoate Cayalyst | 0.06 |
| 10% Calcium Carboxylate | 0.60 |
| Neo Cem 250 | 0.66 |

| | |
|---|---|
| ¹ Irgacure® 500 from Ciba - 50/50 mix of benzophenone/1-hydroxy cyclohexyl phenyl ketone ² Ebercryl® P115 amine synergist from UCB Chemicals Corp. | |

Such a coating could be cured by exposure to high energy such as ultraviolet light and by oxidative curing.

### EXAMPLES 12 -15

Comparative examples 12 - 15 were prepared by mixing the following parts by weight:

| Raw Material | 12 | 13 | 14 | 15 |
|---|---|---|---|---|
| Methacrylated Sucrose Safflower Alkyd of Example 6 | 605.00 | 605.00 | 0.00 | 0.00 |
| Sucrose Safflower Alkyd¹ | 0.00 | 0.00 | 605.00 | 605.00 |
| Mineral Spirits | 6.17 | 6.17 | 0.00 | 0.00 |
| Aromatic 100 Naphtha | 54.29 | 54.29 | 60.46 | 60.46 |
| Bentone SD-2 | 3.00 | 3.00 | 3.00 | 3.00 |
| Soya Lecithin | 14.00 | 14.00 | 14.00 | 14.00 |
| 10% Calcium Carboxylate | 3.00 | 3.00 | 3.00 | 3.00 |
| Titanium Dioxide | 180.00 | 180.00 | 180.00 | 180.00 |
| Aluminum Silicate | 140.00 | 140.00 | 140.00 | 140.00 |
| 12% Cobalt Octoate Catalyst | 1.50 | 1.50 | 1.50 | 1.50 |
| 10% Calcium Carboxylate | 30.00 | 30.00 | 30.00 | 30.00 |
| Methyl Ethyl Ketoxime | 4.00 | 4.00 | 4.00 | 4.00 |
| 10% Photoinitiator Solution² | 0.00 | 60.50 | 0.00 | 60.50 |

| | | | | |
|---|---|---|---|---|
| ¹ Sucrose Safflower Alkyd having an average of about 7.7 of the 8 hydroxyls esterified with the fatty acid. ² Irgacure 819 (bis(2,4,6-trimethylbenzoyl)phenylphosphine oxide) from Ciba Specialty Chemicals dissolved in Oxsol 100 (parachlorobenzotrifluoride) from Kowa America. | | | | |

The coatings of Examples 12 - 15 were applied to a suitable substrate and allowed to cure for 5 hours in sunlight followed by exposure to room light. Examples 12 -15 achieved "dry hard" cure in 14, 12, 72-96, and 72-96, hours respectively.

While this invention has been described by a specific number of embodiments, other variations and modifications may be made without departing from the scope of the invention as set forth in the appended claims.

## Claims

1. An esterified carbohydrate having pendent fatty acid residues and pendent (meth)acrylate groups, wherein the pendent fatty acid residues contain the residues of linoleic, linolenic or oleic acids.

2. The esterified carbohydrate of claim 1 wherein the pendent fatty acid residues have replaced from 3 to about 97% of the total hydroxyl equivalency of the underlying carbohydrate.

3. The esterified carbohydrate of claim 1 wherein the pendent (meth)acrylate groups have replaced from about 3 to about 97% of the total hydroxyl equivalency of the underlying carbohydrate.

4. The esterified carbohydrate of claim 1 wherein the carbohydrate is sucrose.

5. The esterified carbohydrate of claim 1 wherein the carbohydrate is a chain extended carbohydrate.

6. A curable composition comprising:
(i) an esterified carbohydrate as defined in any of claims 1 to 5 having pendent fatty acid residues and pendent (meth)acrylate functionality, wherein the pendent fatty acid residues contain the residues of linoleic, linolenic or oleic acids; and
(ii) at least one drier or initiator for initiating cure.

7. A curable composition comprising:
(i) an esterified carbohydrate as defined in any of claims 1 to 5 having pendent fatty acid residues and pendent (meth)acrylate functionality, wherein the pendent fatty acid residues contain the residues of linoleic, linolenic or oleic acids; and
(ii) at least one other ethylenically unsaturated polymerizable reactant.

8. The curable composition of claim 7 wherein the ethylenically unsaturated polymerizable reactant comprises at least one ethylenically unsaturated monomer.

9. The curable composition of claim 7 wherein the esterified carbohydrate is present at a level of 5% to about 90% of the polymerizable components.

10. The curable composition of claim 7 wherein the composition also comprises an initiator.

11. A curable composition comprising:
(i) an esterified carbohydrate alkyd having pendent fatty acid residues and pendent (meth)acrylate functionality, wherein the pendent fatty acid residues contain the residues of linoleic, linolenic or oleic acids; and
(ii) at least one additional film forming polymer.

12. The composition of claim 11 wherein the film forming polymer is selected from the group consisting of alkyds, polyesters, epoxies, vinyl resins, phenolics, fatty phenolics, acrylics, polyurethanes, polyureas, polyolefins, silicones, silanes, natural oils and mixtures thereof.

13. The composition of claim 12 wherein the additional film forming polymer is an alkyd.

14. The composition of claim 13 wherein the additional film forming polymer is an esterified carbohydrate alkyd having pendent fatty acid residues and which is substantially free of pendent (meth)acrylate functionality.

15. The composition of claim 11 wherein the (meth)acrylate functional esterified carbohydrate is present at a level of at least 5% by weight of all the film forming materials.

16. The composition of claim 15 wherein the (meth)acrylate functional esterified carbohydrate is present at a level of 20 to about 90% by weight of all the film forming materials.

17. A process for producing an esterified carbohydrate as defined in any of claims 1 to 5 having pendent (meth)acrylate groups and pendent fatty acid residues containing linoleic, linolenic or oleic acid residues, said process comprising reacting:
(i) a carbohydrate or reactive derivative thereof; and
(ii) a fatty acid or reactive derivative thereof, wherein the fatty acid contains linoleic, linolenic or oleic acids; and
(iii) a reactant having a (meth)acrylic moiety and a moiety reactive with the carbohydrate or reactive derivative thereof;
and wherein either (ii) or (iii) is reacted first with the carbohydrate or reactive derivative thereof, followed by reaction of the other; or wherein both (ii) and (iii) are reacted with the carbohydrate or reactive derivative thereof at the same time.

18. A coating comprising:
(i) an esterified carbohydrate as defined in any of claims 1 to 5 having pendent fatty acid residues and pendent (meth)acrylate groups, wherein the pendent fatty acid residues contain the residues of linoleic, linolenic or oleic acids; and
(ii) at least one pigment.

19. A process for coating a substrate which process comprises:
(i) applying a coating comprising an esterified carbohydrate as defined in any of claims 1 to 5 having pendent fatty acid residues and pendent (meth)acrylate groups to the substrate, wherein the pendent fatty acid residues contain the residues of linoleic, linolenic or oleic acids;
(ii) curing said coating by exposure to air or to high energy radiation.

## Patentansprüche

1. Ein verestertes Kohlenhydrat mit anhängenden Fettsäureresten und anhängenden (Meth)acrylatgruppen, wobei die anhängenden Fettsäurereste die Reste Linolsäure, Linolensäure oder Ölsäure enthalten.

2. Das veresterte Kohlenhydrat nach Anspruch 1, wobei die anhängenden Fettsäurereste 3 bis etwa 97% der Gesamt-Hydroxyläquivalenz des zugrundeliegenden Kohlenhydrats ersetzt haben.

3. Das veresterte Kohlenhydrat nach Anspruch 1, wobei die anhängenden (Meth)acrylatgruppen etwa 3 bis etwa 97% der Gesamt-Hydroxyläquivalenz des zugrundeliegenden Kohlenhydrats ersetzt haben.

4. Das veresterte Kohlenhydrat nach Anspruch 1, wobei das Kohlenhydrat Saccharose ist.

5. Das veresterte Kohlenhydrat nach Anspruch 1, wobei das Kohlenhydrat ein kettenverlängertes Kohlenhydrat ist.

6. Eine härtbare Zusammensetzung, umfassend:
(i) ein verestertes Kohlenhydrat wie in einem der Ansprüche 1 bis 5 definiert mit anhängenden Fettsäureresten und anhängender (Meth)acrylatfunktionalität, wobei die anhängenden Fettsäurereste die Reste Linolsäure, Linolensäure oder Ölsäure enthalten, und
(ii) wenigstens einen Trockner oder Initiator zur Initiierung der Härtung.

7. Eine härtbare Zusammensetzung, umfassend:
(i) ein verestertes Kohlenhydrat wie in einem der Ansprüche 1 bis 5 definiert mit anhängenden Fettsäureresten und anhängender (Meth)acrylatfunktionalität, wobei die anhängenden Fettsäurereste die Reste Linolsäure, Linolensäure oder Ölsäure enthalten, und
(ii) wenigstens einen anderen ethylenisch ungesättigten polymerisierbaren Reaktant.

8. Die härtbare Zusammensetzung nach Anspruch 7, wobei der ethylenisch ungesättigte polymerisierbare Reaktant wenigstens ein ethylenisch ungesättigtes Monomer umfasst.

9. Die härtbare Zusammensetzung nach Anspruch 7, wobei das veresterte Kohlenhydrat in einer Menge von 5% bis etwa 90% der polymerisierbaren Komponenten vorliegt.

10. Die härtbare Zusammensetzung nach Anspruch 7, wobei die Zusammensetzung auch einen Initiator umfasst.

11. Eine härtbare Zusammensetzung, umfassend:
(i) ein verestertes Kohlenhydrat-Alkydharz mit anhängenden Fettsäureresten und anhängender (Meth)acrylatfunktionalität, wobei die anhängenden Fettsäurereste die Reste Linolsäure, Linolensäure oder Ölsäure enthalten, und
(ii) wenigstens ein zusätzliches filmbildendes Polymer.

12. Die Zusammensetzung nach Anspruch 11, wobei das filmbildende Polymer ausgewählt ist aus der Gruppe bestehend aus Alkydharzen, Polyestern, Epoxiden, Vinylharzen, Phenolharzen, Fettphenolharzen, Acrylharzen, Polyurethanen, Polyharnstoffen, Polyolefinen, Siliconen, Silanen, natürlichen Ölen und Mischungen davon.

13. Die Zusammensetzung nach Anspruch 12, wobei das zusätzliche filmbildende Polymer ein Alkydharz ist.

14. Die Zusammensetzung nach Anspruch 13, wobei das zusätzliche filmbildende Polymer ein verestertes Kohlenhydrat-Alkydharz ist, das anhängende Fettsäurereste besitzt und das im Wesentlichen frei von anhängender (Meth)acrylatfunktionalität ist.

15. Die Zusammensetzung nach Anspruch 11, wobei das (Meth)acrylatfunktionelle veresterte Kohlenhydrat in einer Menge von wenigstens 5 Gew.-%, bezogen auf sämtliche filmbildende Materialien, vorliegt.

16. Die Zusammensetzung nach Anspruch 15, wobei das (Meth)acrylatfunktionelle veresterte Kohlenhydrat in einer Menge von 20 bis etwa 90 Gew.-%, bezogen auf sämtliche filmbildende Materialien, vorliegt.

17. Ein Verfahren zur Erzeugung eines veresterten Kohlenhydrats wie in einem der Ansprüche 1 bis 5 definiert mit anhängenden (Meth)acrylatgruppen und anhängenden Fettsäureresten, die Linolsäure, Linolensäure oder Ölsäure enthalten, wobei das Verfahren die Umsetzung von:
(i) einem Kohlenhydrat oder einem reaktiven Derivat davon und
(ii) einer Fettsäure oder einem reaktiven Derivat davon, wobei die Fettsäure Linolsäure, Linolensäure oder Ölsäure enthält, und
(ii) einem Reaktant mit einem (Meth)acrylrest und einem mit dem Kohlenhydrat oder dem reaktiven Derivat davon reaktiven Rest
umfasst, und wobei entweder (ii) oder (iii) zuerst mit dem Kohlenhydrat oder dem reaktiven Derivat davon umgesetzt wird, gefolgt von der Umsetzung des Anderen, oder wobei (ii) und (iii) gleichzeitig mit dem Kohlenhydrat oder dem reaktiven Derivat davon umgesetzt werden.

18. Eine Beschichtung, umfassend:
(i) ein verestertes Kohlenhydrat wie in einem der Ansprüche 1 bis 5 definiert mit anhängenden Fettsäureresten und anhängenden (Meth)acylatgruppen, wobei die anhängenden Fettsäurereste die Reste Linolsäure, Linolensäure oder Ölsäure enthalten, und
(ii) wenigstens ein Pigment.

19. Ein Verfahren zur Beschichtung eines Substrats, wobei das Verfahren umfasst:
(i) Auftragen einer Beschichtung, umfassend ein verestertes Kohlenhydrat wie in einem der Ansprüche 1 bis 5 definiert mit anhängenden Fettsäureresten und anhängenden (Meth)acylatgruppen, wobei die anhängenden Fettsäurereste die Reste Linolsäure, Linolensäure oder Ölsäure enthalten, auf das Substrat,
(ii) Aushärten der Beschichtung durch Einwirkung von Luft oder energiereicher Strahlung.

## Revendications

1. Hydrate de carbone estérifié ayant des résidus acides gras pendants et des groupements (méth)acrylates pendants, où les résidus acides gras pendants contiennent les résidus des acides linoléique, linolénique ou oléique.

2. Hydrate de carbone estérifié de la revendication 1, où les résidus acides gras pendants ont remplacé de 3 à environ 97 % de l'équivalence hydroxyle totale de l'hydrate de carbone de base.

3. Hydrate de carbone estérifié de la revendication 1, où les groupements (méth)acrylates pendants ont remplacé d'environ 3 à environ 97 % de l'équivalence hydroxyle totale de l'hydrate de carbone de base.

4. Hydrate de carbone estérifié de la revendication 1, où l'hydrate de carbone est le sucrose.

5. Hydrate de carbone estérifié de la revendication 1, où l'hydrate de carbone est un hydrate de carbone à chaîne allongée.

6. Composition durcissable comprenant :
(i) un hydrate de carbone estérifié tel que défini à l'une quelconque des revendications 1 à 5 ayant des résidus acides gras pendants et une fonctionnalité (méth)acrylate pendante, où les résidus acides gras pendants contiennent les résidus des acides linoléique, linolénique ou oléique ; et
(ii) au moins un dessiccateur ou un amorceur pour amorcer le durcissement.

7. Composition durcissable comprenant :
(i) un hydrate de carbone estérifié tel que défini à l'une quelconque des revendications 1 à 5 ayant des résidus acides gras pendants et une fonctionnalité (méth)acrylate pendante, où les résidus acides gras pendants contiennent les résidus des acides linoléique, linolénique ou oléique ; et
(ii) au moins un autre réactif à insaturation éthylénique polymérisable.

8. Composition durcissable de la revendication 7, où le réactif à insaturation éthylénique polymérisable comprend au moins un monomère à insaturation éthylénique.

9. Composition durcissable de la revendication 7, où l'hydrate de carbone estérifié est présent à un taux qui représente de 5 % à environ 90 % des composants polymérisables.

10. Composition durcissable de la revendication 7, où la composition comprend également un amorceur.

11. Composition durcissable comprenant :
(i) alkyde hydrocarboné estérifié ayant des résidus acides gras pendants et une fonctionnalité (méth)acrylate pendante, où les résidus acides gras pendants contiennent les résidus des acides linoléique, linolénique ou oléique ; et
(ii) au moins un polymère filmogène additionnel.

12. Composition de la revendication 11, où le polymère filmogène est sélectionné dans le groupe consistant en des alkydes, polyesters, époxydes, résines vinyliques, composés phénoliques, composés phénoliques gras, acryliques, polyuréthanes, polyurées, polyoléfines, silicones, silanes, huiles naturelles et des mélanges de ceux-ci.

13. Composition de la revendication 12, où le polymère filmogène additionnel est un alkyde.

14. Composition de la revendication 13, où le polymère filmogène additionnel est un alkyde hydrocarboné estérifié ayant des résidus acide gras pendants qui est essentiellement exempt de fonctionnalités (méth)acrylates pendantes.

15. Composition de la revendication 11, où l'hydrate de carbone estérifié à fonctionnalité (méth)acrylate est présent à un taux qui représente au moins 5 % en poids de l'ensemble des matériaux filmogènes.

16. Composition de la revendication 15, où l'hydrate de carbone estérifié à fonctionnalité (méth)acrylate est présent à un taux qui représente de 20 à environ 90 % en poids de l'ensemble des matériaux filmogènes.

17. Procédé de production d'un hydrate de carbone estérifié tel que défini à l'une quelconque des revendications 1 à 5 ayant des groupements (méth)acrylates pendants et des résidus acides gras pendants contenant des résidus des acides linoléique, linolénique ou oléique, ledit procédé comprenant la mise en réaction :
(i) d'un hydrate de carbone ou d'un dérivé réactif de celui-ci ; et
(ii) d'un acide gras ou d'un dérivé réactif de celui-ci, où l'acide gras contient des acides linoléique, linolénique ou oléique ; et
(iii) un réactif ayant une fraction (méth)acrylique et une fraction capable de réagir avec l'hydrate de carbone ou le dérivé réactif de celui-ci ;
et où (ii) ou (iii) est en premier lieu mis en réaction avec l'hydrate de carbone ou le dérivé réactif de celui-ci avant la mise en réaction de l'autre ; ou où (ii) et (iii) sont mis en réaction en même temps avec l'hydrate de carbone ou le dérivé réactif de celui-ci.

18. Revêtement comprenant:
(i) un hydrate de carbone estérifié tel que défini à l'une quelconque des revendications 1 à 5 ayant des résidus acides gras pendants et des groupements (méth)acrylates pendants, où les résidus acides gras pendants contiennent les résidus des acides linoléique, linolénique ou oléique ; et
(ii) au moins un pigment.

19. Procédé de revêtement d'un substrat, ledit procédé comprenant :
(i) l'application sur un substrat d'un revêtement comprenant un hydrate de carbone estérifié tel que défini à l'une quelconque des revendications 1 à 5 ayant des résidus acides gras pendants et des groupements (méth)acrylates pendants, où les résidus acides gras pendants contiennent les résidus des acides linoléique, linolénique ou oléique ;
(ii) le durcissement dudit revêtement par exposition à l'air ou à un rayonnement de haute énergie.
